# EUROPEAN PATENT SPECIFICATION

(11) **EP 2 598 070 B1**
(45) Date of publication and mention of the grant of the patent: **21.12.2016**
(21) Application number: 11740794.0
(22) Date of filing: 29.07.2011
(51) Int. Cl.: A61B 18/14, A61B 17/00, A61B 18/02, A61B 18/00

(54) **BALLOON WITH SURFACE ELECTRODES AND INTEGRAL COOLING FOR RENAL NERVE ABLATION**
BALLON MIT OBERFLÄCHENELEKTRODEN UND INTEGRIERTER KÜHLUNG FÜR NIERENNERVENABLATION
BALLONNET AVEC ÉLECTRODES DE SURFACE ET REFROIDISSEMENT INTÉGRAL POUR ABLATION DE NERF RÉNAL

(30) Priority: 28.07.2011 US 201113193338; 30.07.2010 US 369453 P
(43) Date of publication of application: 05.06.2013
(73) Proprietor: Boston Scientific Scimed, Inc., Maple Grove, MN 55311 (US)
(72) Inventor: WILLARD, Martin, R., Burnsville MN 55337 (US); KOBLISH, Joe, Sunnyvale CA 94087 (US); HASTINGS, Roger, Maple Grove MN 55369 (US)
(74) Representative: Vossius & Partner Patentanwälte Rechtsanwälte mbB
(86) International application number: PCT/US2011/045879
(87) International publication number: WO 2012/016135

(56) References cited:
- WO-A1-00/69376
- WO-A1-97/32532
- WO-A1-2009/137819
- WO-A1-2011/060200
- WO-A2-2007/121309
- WO-A2-2007/146834
- WO-A2-2008/003058
- WO-A2-2011/056684
- US-A1- 2007 129 761
- US-A1- 2007 135 875
- US-A1- 2007 265 687
- US-A1- 2009 299 356

## Description

### BACKGROUND

WO-A-0 069 376 discloses a catheter, lumen, a balloon carrying electrodes and thermocouples that are used for feedback control, as well as a cooling arrangement using a cooling liquid.

WO-A-2009 137 819 discloses a system comprising a catheter, a balloon made of a thermally conductive material carrying one or more electrodes and a cooling arrangement, as well as a temperature sensor used for feedback control.

### SUMMARY

Embodiments of the disclosure are directed to ablating target tissue of the body, such as innervated renal tissue, using an intravascular ablation device with integral cooling. Embodiments of the disclosure are directed to systems, apparatuses, and methods for ablating target tissue of the body, such as innervated renal tissue, using balloon supported ablation electrodes and an integral cooling arrangement for cooling the ablation electrodes.

The invention provides an apparatus according to claim 1. Preferred embodiments are defined in the dependent claims.

Any embodiments, examples and aspects disclosed herein, but not falling under the scope of claim 1, do not form part of the invention. This applies in particular to the methods disclosed herein.

### BRIEF DESCRIPTION OF THE DRAWINGS

Figure 1 is an illustration of a right kidney and renal vasculature including a renal artery branching laterally from the abdominal aorta;
Figures 2A and 2B illustrate sympathetic innervation of the renal artery;
Figure 3A illustrates various tissue layers of the wall of the renal artery;
Figures 3B and 3C illustrate a portion of a renal nerve;
Figure 4 shows a therapy device of an ablation catheter which includes a cooling arrangement and balloon supported ablation electrodes in accordance with various embodiments;
Figure 5 shows a therapy device of an ablation catheter which includes a cooling arrangement and balloon supported ablation electrodes in accordance with various embodiments;
Figure 6 is a cross-sectional view of a portion of a therapy device showing an electrode-tissue interface defined between a balloon supported ablation electrode and a wall of a renal artery in accordance with various embodiments;
Figure 7 is a cross-sectional view of a portion of a therapy device showing an electrode-tissue interface defined between a balloon supported ablation electrode and a wall of a renal artery in accordance with various embodiments;
Figures 8-10 show features of a cooling arrangement for a portion of a therapy device which includes a balloon supported ablation electrode in accordance with various embodiments; and
Figure 11 shows a therapy system configured to perform renal denervation using a therapy device of an ablation catheter which includes a cooling arrangement and balloon supported ablation electrodes in accordance with various embodiments.

### DESCRIPTION

Embodiments of the disclosure are directed to apparatuses and methods for ablating target tissue using electrical energy delivered by a multiplicity of cooled ablation electrodes supported by an expandable therapy device. Embodiments of the disclosure are directed to apparatuses and methods for ablating target tissue located adjacent to a body vessel using a multiplicity of cooled ablation electrodes supported by an expandable therapy device deployed in the body vessel. Embodiments are directed to ablating target tissue of the body using cooled ablation electrodes situated at a wall of the target vessel proximate the target tissue, such that the cooled ablation electrodes translate a location at which steady-state ablative heating begins from an electrode-tissue interface at the target vessel wall to a desired location a predetermined distance away from the electrode-tissue interface. Particular embodiments of the disclosure are directed to apparatuses and methods for ablating perivascular renal nerves for the treatment of hypertension.

Radiofrequency (RF) ablation of renal nerves, which lie proximate to the adventitia of the renal artery, may be an effective treatment for chronic hypertension. It has been difficult to effectively ablate perivascular renal sympathetic nerves by access from the renal artery, without injury to the renal artery wall. To reduce concern for potential stenotic narrowing of the artery after the ablation procedure, minimizing arterial injury during such an ablation procedure is important.

Embodiments of the disclosure incorporate a housing mounted at a distal end of a therapy catheter for supporting ablation electrodes and cooling components of the therapy device. The housing encompasses at least a portion of a cooling arrangement and supports a number of ablation electrodes on an outer surface of the housing. The housing is preferably transformable between a low-profile introduction configuration and a larger-profile deployment configuration. The low-profile introduction configuration allows the therapy catheter to be readily advanced through the venous or arterial system to a desired body location, for example. The larger-profile deployed configuration allows the therapy catheter to be stabilized at the desired body location, such as within the renal artery. In various embodiments, the expandable structure comprises a balloon, such as a cooling balloon or a cryoballoon.

Various embodiments of the disclosure include a balloon catheter with electrodes on the balloon to perform ablation of target tissue while cooling the luminal surface of a renal artery prevents undesirable heating of non-targeted tissue of the renal artery, particularly the endothelium of the artery. Apparatuses of the disclosure can provide a number of benefits, including one or more of reduced injury to the artery, ablation with a single treatment rather than multiple treatments which reduces treatment time, and ablation in a manner that is more controllable and repeatable.

An RF electrode can be cooled to limit temperature increase at the electrode surface while allowing increased temperature at a distance from the electrode. When the electrode is in contact with tissue, the distance where steady-state heating starts is preferably on the order of about 0.5 mm to about 1 mm into the tissue. For example, it is desirable that steady-state ablative heating begins at a distance of at least about 0.5 mm away from the electrode surface. Heat is conducted out from that point. In a blood vessel, limiting heat at the electrode-vessel surface (also referred to herein as an electrode-tissue interface) can limit injury at the vessel surface, which can reduce thermal injury to, and yield improved healing of, the vessel surface. One or more temperature sensors, such as thermocouples, can be provided at the site of the electrodes to measure the temperature at or proximate the electrodes. In some embodiments, a temperature sensor is positioned near or at the site of each electrode on the balloon, allowing for precision temperature measurements at individual electrode locations of the ablation electrode arrangement.

Cooling of the electrodes, and other portions of the balloon wall if desired, can be effected using several different cooling mechanisms. In some embodiments, a therapy catheter incorporates a phase-change cryothermal capability such as by spraying a cryogen to cool at least the electrode supporting portions of an inflated balloon. Temperature and/or pressure sensors or other sensor elements (e.g., impedance sensors) can be incorporated near or at the electrode locations or other locations to facilitate monitoring and control of the ablation procedure. In other embodiments, the therapy catheter incorporates a heat exchange apparatus configured to receive a liquid coolant capable of causing freezing of tissue proximate of the target tissue, such as the wall of the renal artery. In some embodiments, the therapy catheter incorporates one or more solid-state thermoelectric cooling devices, such as Peltier devices. The cooling and ablation electrode components of the therapy catheter can interface with external control units to control device functioning and monitor or display temperatures, power used, impedance, blood pressure, or other parameters.

Various embodiments of the disclosure are directed to apparatuses and methods for renal denervation for treating hypertension. Hypertension is a chronic medical condition in which the blood pressure is elevated. Persistent hypertension is a significant risk factor associated with a variety of adverse medical conditions, including heart attacks, heart failure, arterial aneurysms, and strokes. Persistent hypertension is a leading cause of chronic renal failure. Hyperactivity of the sympathetic nervous system serving the kidneys is associated with hypertension and its progression. Deactivation of nerves in the kidneys via renal denervation can reduce blood pressure, and may be a viable treatment option for many patients with hypertension who do not respond to conventional drugs.

The kidneys are instrumental in a number of body processes, including blood filtration, regulation of fluid balance, blood pressure control, electrolyte balance, and hormone production. One primary function of the kidneys is to remove toxins, mineral salts, and water from the blood to form urine. The kidneys receive about 20-25% of cardiac output through the renal arteries that branch left and right from the abdominal aorta, entering each kidney at the concave surface of the kidneys, the renal hilum.

Blood flows into the kidneys through the renal artery and the afferent arteriole, entering the filtration portion of the kidney, the renal corpuscle. The renal corpuscle is composed of the glomerulus, a thicket of capillaries, surrounded by a fluid-filled, cup-like sac called Bowman's capsule. Solutes in the blood are filtered through the very thin capillary walls of the glomerulus due to the pressure gradient that exists between the blood in the capillaries and the fluid in the Bowman's capsule. The pressure gradient is controlled by the contraction or dilation of the arterioles. After filtration occurs, the filtered blood moves through the efferent arteriole and the peritubular capillaries, converging in the interlobular veins, and finally exiting the kidney through the renal vein.

Particles and fluid filtered from the blood move from the Bowman's capsule through a number of tubules to a collecting duct. Urine is formed in the collecting duct and then exits through the ureter and bladder. The tubules are surrounded by the peritubular capillaries (containing the filtered blood). As the filtrate moves through the tubules and toward the collecting duct, nutrients, water, and electrolytes, such as sodium and chloride, are reabsorbed into the blood.

The kidneys are innervated by the renal plexus which emanates primarily from the aorticorenal ganglion. Renal ganglia are formed by the nerves of the renal plexus as the nerves follow along the course of the renal artery and into the kidney. The renal nerves are part of the autonomic nervous system which includes sympathetic and parasympathetic components. The sympathetic nervous system is known to be the system that provides the bodies "fight or flight" response, whereas the parasympathetic nervous system provides the "rest and digest" response. Stimulation of sympathetic nerve activity triggers the sympathetic response which causes the kidneys to increase production of hormones that increase vasoconstriction and fluid retention. This process is referred to as the renin-angiotensin-aldosterone-system (RAAS) response to increased renal sympathetic nerve activity.

In response to a reduction in blood volume, the kidneys secrete renin, which stimulates the production of angiotensin. Angiotensin causes blood vessels to constrict, resulting in increased blood pressure, and also stimulates the secretion of the hormone aldosterone from the adrenal cortex. Aldosterone causes the tubules of the kidneys to increase the reabsorption of sodium and water, which increases the volume of fluid in the body and blood pressure.

Congestive heart failure (CHF) is a condition that has been linked to kidney function. CHF occurs when the heart is unable to pump blood effectively throughout the body. When blood flow drops, renal function degrades because of insufficient perfusion of the blood within the renal corpuscles. The decreased blood flow to the kidneys triggers an increase in sympathetic nervous system activity (i.e., the RAAS becomes too active) that causes the kidneys to secrete hormones that increase fluid retention and vasorestriction. Fluid retention and vasorestriction in turn increases the peripheral resistance of the circulatory system, placing an even greater load on the heart, which diminishes blood flow further. If the deterioration in cardiac and renal functioning continues, eventually the body becomes overwhelmed, and an episode of heart failure decompensation occurs, often leading to hospitalization of the patient.

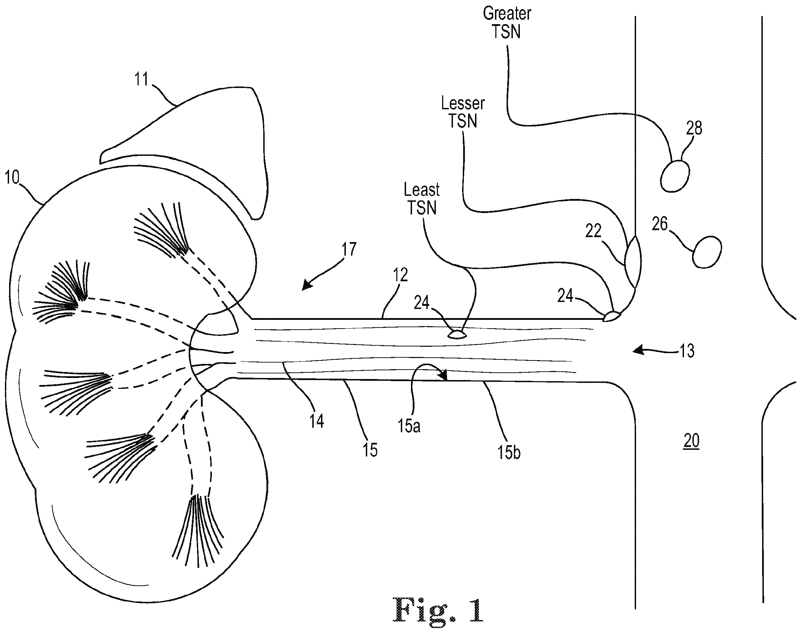

Figure 1 is an illustration of a right kidney 10 and renal vasculature including a renal artery 12 branching laterally from the abdominal aorta 20. In Figure 1, only the right kidney 10 is shown for purposes of simplicity of explanation, but reference will be made herein to both right and left kidneys and associated renal vasculature and nervous system structures, all of which are contemplated within the context of embodiments of the disclosure. The renal artery 12 is purposefully shown to be disproportionately larger than the right kidney 10 and abdominal aorta 20 in order to facilitate discussion of various features and embodiments of the present disclosure.

The right and left kidneys are supplied with blood from the right and left renal arteries that branch from respective right and left lateral surfaces of the abdominal aorta 20. Each of the right and left renal arteries is directed across the crus of the diaphragm, so as to form nearly a right angle with the abdominal aorta 20. The right and left renal arteries extend generally from the abdominal aorta 20 to respective renal sinuses proximate the hilum 17 of the kidneys, and branch into segmental arteries and then interlobular arteries within the kidney 10. The interlobular arteries radiate outward, penetrating the renal capsule and extending through the renal columns between the renal pyramids. Typically, the kidneys receive about 20% of total cardiac output which, for normal persons, represents about 1200 mL of blood flow through the kidneys per minute.

The primary function of the kidneys is to maintain water and electrolyte balance for the body by controlling the production and concentration of urine. In producing urine, the kidneys excrete wastes such as urea and ammonium. The kidneys also control reabsorption of glucose and amino acids, and are important in the production of hormones including vitamin D, renin and erythropoietin.

An important secondary function of the kidneys is to control metabolic homeostasis of the body. Controlling hemostatic functions include regulating electrolytes, acid-base balance, and blood pressure. For example, the kidneys are responsible for regulating blood volume and pressure by adjusting volume of water lost in the urine and releasing erythropoietin and renin, for example. The kidneys also regulate plasma ion concentrations (e.g., sodium, potassium, chloride ions, and calcium ion levels) by controlling the quantities lost in the urine and the synthesis of calcitrol. Other hemostatic functions controlled by the kidneys include stabilizing blood pH by controlling loss of hydrogen and bicarbonate ions in the urine, conserving valuable nutrients by preventing their excretion, and assisting the liver with detoxification.

Also shown in Figure 1 is the right suprarenal gland 11, commonly referred to as the right adrenal gland. The suprarenal gland 11 is a star-shaped endocrine gland that rests on top of the kidney 10. The primary function of the suprarenal glands (left and right) is to regulate the stress response of the body through the synthesis of corticosteroids and catecholamines, including cortisol and adrenaline (epinephrine), respectively. Encompassing the kidneys 10, suprarenal glands 11, renal vessels 12, and adjacent perirenal fat is the renal fascia, e.g., Gerota's fascia, (not shown), which is a fascial pouch derived from extraperitoneal connective tissue.

The autonomic nervous system of the body controls involuntary actions of the smooth muscles in blood vessels, the digestive system, heart, and glands. The autonomic nervous system is divided into the sympathetic nervous system and the parasympathetic nervous system. In general terms, the parasympathetic nervous system prepares the body for rest by lowering heart rate, lowering blood pressure, and stimulating digestion. The sympathetic nervous system effectuates the body's fight-or-flight response by increasing heart rate, increasing blood pressure, and increasing metabolism.

In the autonomic nervous system, fibers originating from the central nervous system and extending to the various ganglia are referred to as preganglionic fibers, while those extending from the ganglia to the effector organ are referred to as postganglionic fibers. Activation of the sympathetic nervous system is effected through the release of adrenaline (epinephrine) and to a lesser extent norepinephrine from the suprarenal glands 11. This release of adrenaline is triggered by the neurotransmitter acetylcholine released from preganglionic sympathetic nerves.

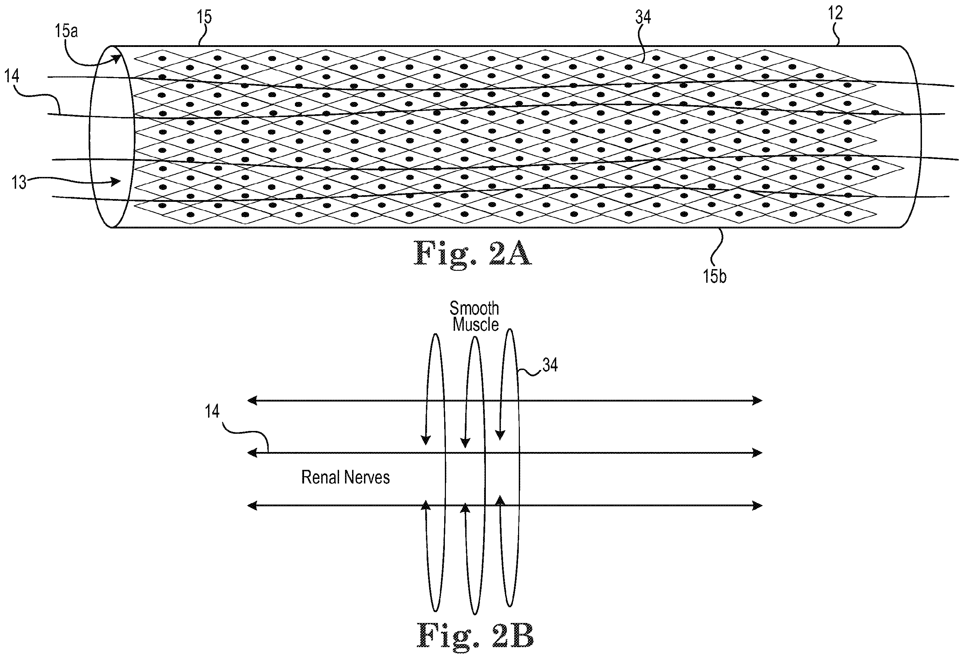

The kidneys and ureters (not shown) are innervated by the renal nerves 14. Figures 1 and 2A-2B illustrate sympathetic innervation of the renal vasculature, primarily innervation of the renal artery 12. The primary functions of sympathetic innervation of the renal vasculature include regulation of renal blood flow and pressure, stimulation of renin release, and direct stimulation of water and sodium ion reabsorption.

Most of the nerves innervating the renal vasculature are sympathetic postganglionic fibers arising from the superior mesenteric ganglion 26. The renal nerves 14 extend generally axially along the renal arteries 12, enter the kidneys 10 at the hilum 17, follow the branches of the renal arteries 12 within the kidney 10, and extend to individual nephrons. Other renal ganglia, such as the renal ganglia 24, superior mesenteric ganglion 26, the left and right aorticorenal ganglia 22, and celiac ganglia 28 also innervate the renal vasculature. The celiac ganglion 28 is joined by the greater thoracic splanchnic nerve (greater TSN). The aorticorenal ganglia 26 is joined by the lesser thoracic splanchnic nerve (lesser TSN) and innervates the greater part of the renal plexus.

Sympathetic signals to the kidney 10 are communicated via innervated renal vasculature that originates primarily at spinal segments T10-T12 and L1. Parasympathetic signals originate primarily at spinal segments S2-S4 and from the medulla oblongata of the lower brain. Sympathetic nerve traffic travels through the sympathetic trunk ganglia, where some may synapse, while others synapse at the aorticorenal ganglion 22 (via the lesser thoracic splanchnic nerve, i.e., lesser TSN) and the renal ganglion 24 (via the least thoracic splanchnic nerve, i.e., least TSN). The postsynaptic sympathetic signals then travel along nerves 14 of the renal artery 12 to the kidney 10. Presynaptic parasympathetic signals travel to sites near the kidney 10 before they synapse on or near the kidney 10.

With particular reference to Figure 2A, the renal artery 12, as with most arteries and arterioles, is lined with smooth muscle 34 that controls the diameter of the renal artery lumen 13. Smooth muscle, in general, is an involuntary non-striated muscle found within the media layer of large and small arteries and veins, as well as various organs. The glomeruli of the kidneys, for example, contain a smooth muscle-like cell called the mesangial cell. Smooth muscle is fundamentally different from skeletal muscle and cardiac muscle in terms of structure, function, excitation-contraction coupling, and mechanism of contraction.

Smooth muscle cells can be stimulated to contract or relax by the autonomic nervous system, but can also react on stimuli from neighboring cells and in response to hormones and blood borne electrolytes and agents (e.g., vasodilators or vasoconstrictors). Specialized smooth muscle cells within the afferent arteriole of the juxtaglomerular apparatus of kidney 10, for example, produces renin which activates the angiotension II system.

The renal nerves 14 innervate the smooth muscle 34 of the renal artery wall 15 and extend lengthwise in a generally axial or longitudinal manner along the renal artery wall 15. The smooth muscle 34 surrounds the renal artery circumferentially, and extends lengthwise in a direction generally transverse to the longitudinal orientation of the renal nerves 14, as is depicted in Figure 2B.

The smooth muscle 34 of the renal artery 12 is under involuntary control of the autonomic nervous system. An increase in sympathetic activity, for example, tends to contract the smooth muscle 34, which reduces the diameter of the renal artery lumen 13 and decreases blood perfusion. A decrease in sympathetic activity tends to cause the smooth muscle 34 to relax, resulting in vessel dilation and an increase in the renal artery lumen diameter and blood perfusion. Conversely, increased parasympathetic activity tends to relax the smooth muscle 34, while decreased parasympathetic activity tends to cause smooth muscle contraction.

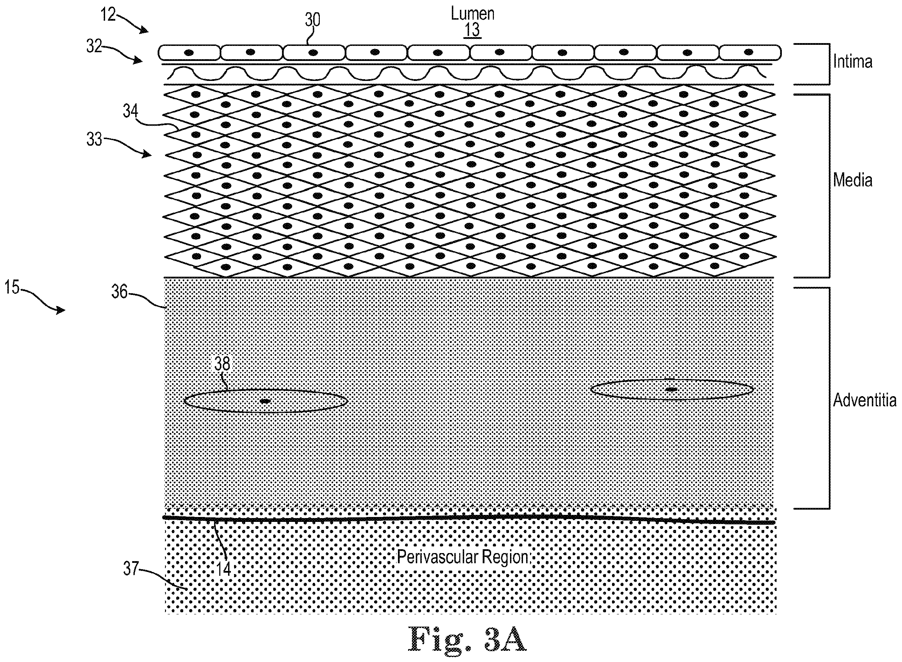

Figure 3A shows a segment of a longitudinal cross-section through a renal artery, and illustrates various tissue layers of the wall 15 of the renal artery 12. The innermost layer of the renal artery 12 is the endothelium 30, which is the innermost layer of the intima 32 and is supported by an internal elastic membrane. The endothelium 30 is a single layer of cells that contacts the blood flowing though the vessel lumen 13. Endothelium cells are typically polygonal, oval, or fusiform, and have very distinct round or oval nuclei. Cells of the endothelium 30 are involved in several vascular functions, including control of blood pressure by way of vasoconstriction and vasodilation, blood clotting, and acting as a barrier layer between contents within the lumen 13 and surrounding tissue, such as the membrane of the intima 32 separating the intima 32 from the media 34, and the adventitia 36. The membrane or maceration of the intima 32 is a fine, transparent, colorless structure which is highly elastic, and commonly has a longitudinal corrugated pattern.

Adjacent the intima 32 is the media 33, which is the middle layer of the renal artery 12. The media is made up of smooth muscle 34 and elastic tissue. The media 33 can be readily identified by its color and by the transverse arrangement of its fibers. More particularly, the media 33 consists principally of bundles of smooth muscle fibers 34 arranged in a thin plate-like manner or lamellae and disposed circularly around the arterial wall 15. The outermost layer of the renal artery wall 15 is the adventitia 36, which is made up of connective tissue. The adventitia 36 includes fibroblast cells 38 that play an important role in wound healing.

A perivascular region 37 is shown adjacent and peripheral to the adventitia 36 of the renal artery wall 15. A renal nerve 14 is shown proximate the adventitia 36 and passing through a portion of the perivascular region 37. The renal nerve 14 is shown extending substantially longitudinally along the outer wall 15 of the renal artery 12. The main trunk of the renal nerves 14 generally lies in or on the adventitia 36 of the renal artery 12, often passing through the perivascular region 37, with certain branches coursing into the media 33 to enervate the renal artery smooth muscle 34.

Embodiments of the disclosure may be implemented to provide varying degrees of denervation therapy to innervated renal vasculature. For example, embodiments of the disclosure may provide for control of the extent and relative permanency of renal nerve impulse transmission interruption achieved by denervation therapy delivered using a treatment apparatus of the disclosure. The extent and relative permanency of renal nerve injury may be tailored to achieve a desired reduction in sympathetic nerve activity (including a partial or complete block) and to achieve a desired degree of permanency (including temporary or irreversible injury).

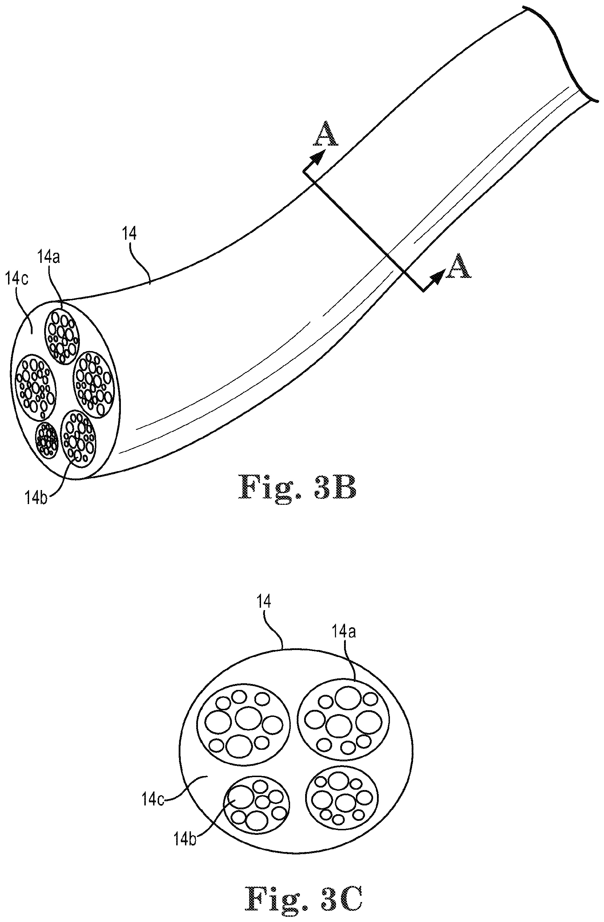

Returning to Figures 3B and 3C, the portion of the renal nerve 14 shown in Figures 3B and 3C includes bundles 14a of nerve fibers 14b each comprising axons or dendrites that originate or terminate on cell bodies or neurons located in ganglia or on the spinal cord, or in the brain. Supporting tissue structures 14c of the nerve 14 include the endoneurium (surrounding nerve axon fibers), perineurium (surrounds fiber groups to form a fascicle), and epineurium (binds fascicles into nerves), which serve to separate and support nerve fibers 14b and bundles 14a. In particular, the endoneurium, also referred to as the endoneurium tube or tubule, is a layer of delicate connective tissue that encloses the myelin sheath of a nerve fiber 14b within a fasciculus.

Major components of a neuron include the soma, which is the central part of the neuron that includes the nucleus, cellular extensions called dendrites, and axons, which are cable-like projections that carry nerve signals. The axon terminal contains synapses, which are specialized structures where neurotransmitter chemicals are released in order to communicate with target tissues. The axons of many neurons of the peripheral nervous system are sheathed in myelin, which is formed by a type of glial cell known as Schwann cells. The myelinating Schwann cells are wrapped around the axon, leaving the axolemma relatively uncovered at regularly spaced nodes, called nodes of Ranvier. Myelination of axons enables an especially rapid mode of electrical impulse propagation called saltation.

In some embodiments, a treatment apparatus of the disclosure may be implemented to deliver denervation therapy that causes transient and reversible injury to renal nerve fibers 14b. In other embodiments, a treatment apparatus of the disclosure may be implemented to deliver denervation therapy that causes more severe injury to renal nerve fibers 14b, which may be reversible if the therapy is terminated in a timely manner. In preferred embodiments, a treatment apparatus of the disclosure may be implemented to deliver denervation therapy that causes severe and irreversible injury to renal nerve fibers 14b, resulting in permanent cessation of renal sympathetic nerve activity. For example, a treatment apparatus may be implemented to deliver a denervation therapy that disrupts nerve fiber morphology to a degree sufficient to physically separate the endoneurium tube of the nerve fiber 14b, which can prevent regeneration and re-innervation processes.

By way of example, and in accordance with Seddon's classification as is known in the art, a treatment apparatus of the disclosure may be implemented to deliver a denervation therapy that interrupts conduction of nerve impulses along the renal nerve fibers 14b by imparting damage to the renal nerve fibers 14b consistent with neruapraxia. Neurapraxia describes nerve damage in which there is no disruption of the nerve fiber 14b or its sheath. In this case, there is an interruption in conduction of the nerve impulse down the nerve fiber, with recovery taking place within hours to months without true regeneration, as Wallerian degeneration does not occur. Wallerian degeneration refers to a process in which the part of the axon separated from the neuron's cell nucleus degenerates. This process is also known as anterograde degeneration. Neurapraxia is the mildest form of nerve injury that may be imparted to renal nerve fibers 14b by use of a treatment apparatus according to embodiments of the disclosure.

A treatment apparatus may be implemented to interrupt conduction of nerve impulses along the renal nerve fibers 14b by imparting damage to the renal nerve fibers consistent with axonotmesis. Axonotmesis involves loss of the relative continuity of the axon of a nerve fiber and its covering of myelin, but preservation of the connective tissue framework of the nerve fiber. In this case, the encapsulating support tissue 14c of the nerve fiber 14b are preserved. Because axonal continuity is lost, Wallerian degeneration occurs. Recovery from axonotmesis occurs only through regeneration of the axons, a process requiring time on the order of several weeks or months. Electrically, the nerve fiber 14b shows rapid and complete degeneration. Regeneration and re-innervation may occur as long as the endoneural tubes are intact.

A treatment apparatus may be implemented to interrupt conduction of nerve impulses along the renal nerve fibers 14b by imparting damage to the renal nerve fibers 14b consistent with neurotmesis. Neurotmesis, according to Seddon's classification, is the most serious nerve injury in the scheme. In this type of injury, both the nerve fiber 14b and the nerve sheath are disrupted. While partial recovery may occur, complete recovery is not possible. Neurotmesis involves loss of continuity of the axon and the encapsulating connective tissue 14c, resulting in a complete loss of autonomic function, in the case of renal nerve fibers 14b. If the nerve fiber 14b has been completely divided, axonal regeneration causes a neuroma to form in the proximal stump.

A more stratified classification of neurotmesis nerve damage may be found by reference to the Sunderland System as is known in the art. The Sunderland System defines five degrees of nerve damage, the first two of which correspond closely with neurapraxia and axonotmesis of Seddon's classification. The latter three Sunderland System classifications describe different levels of neurotmesis nerve damage.

The first and second degrees of nerve injury in the Sunderland system are analogous to Seddon's neurapraxia and axonotmesis, respectively. Third degree nerve injury, according to the Sunderland System, involves disruption of the endoneurium, with the epineurium and perineurium remaining intact. Recovery may range from poor to complete depending on the degree of intrafascicular fibrosis. A fourth degree nerve injury involves interruption of all neural and supporting elements, with the epineurium remaining intact. The nerve is usually enlarged. Fifth degree nerve injury involves complete transection of the nerve fiber 14b with loss of continuity.

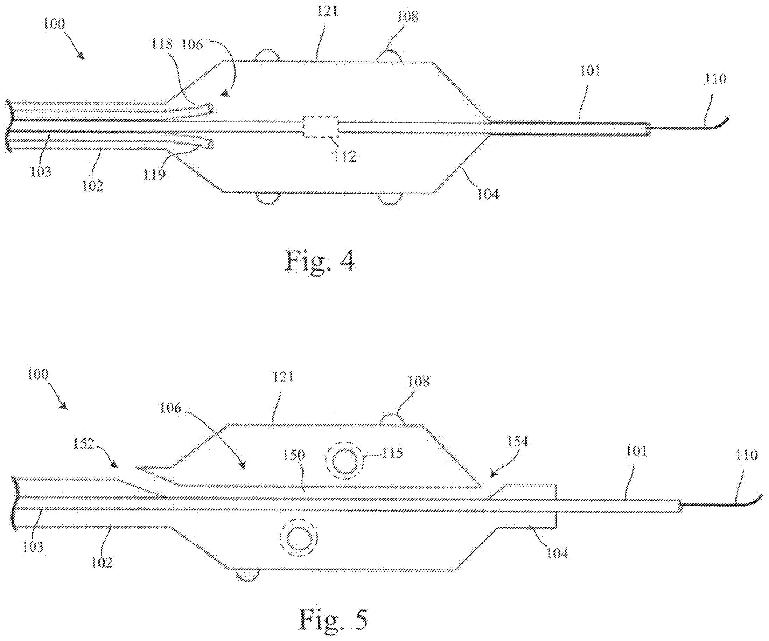

Figure 4 shows an embodiment of the disclosure which includes a therapy catheter 100 configured for placement within a lumen of a target vessel of the body, such as patient's renal artery. The therapy catheter 100 shown in Figure 4 includes a therapy device 104 provided at a distal end of a shaft 102 of the therapy catheter 100. The therapy device 104 includes a multiplicity of electrodes 108 supported by an expandable housing 121 and configured to deliver ablative electrical energy (e.g., RF energy or other form of high frequency AC energy) to target tissue located adjacent the target vessel. The therapy device 104 further includes a cooling arrangement 106 configured to cool each of the electrodes 108 and, if desired, other portions of a wall of the housing 121.

During ablation, the electrodes 108 are cooled by the cooling arrangement 106 such that a location at which steady-state ablative heating begins is translated from an electrode-tissue interface to a location a predetermined distance away from the electrode-tissue interface. Translating the location at which study-state ablative heating begins away from the electrode-tissue interface provides for effective ablating of target tissue while intervening target vessel wall tissue is thermally protected.

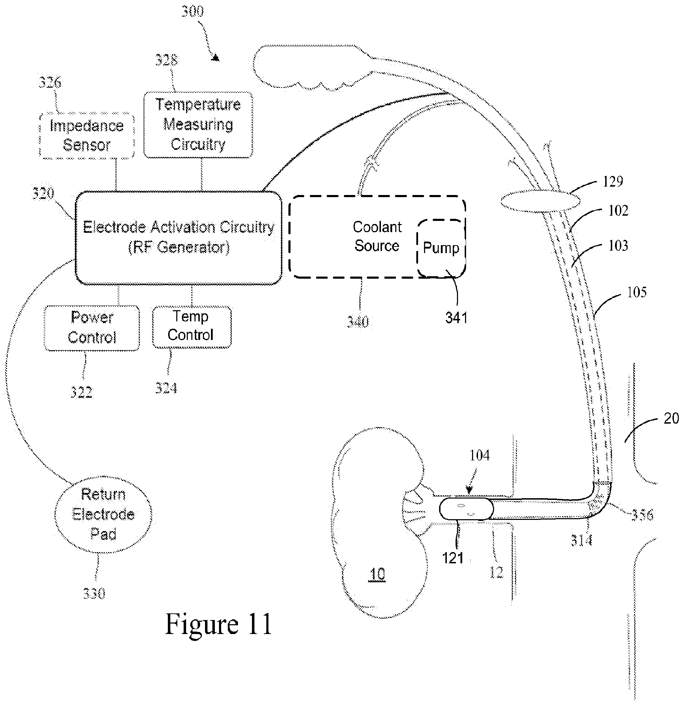

As is further shown in Figure 4, the therapy device 104 is fluidly and electrically coupled to a lumen arrangement 103 which runs along the length of the shaft 102. The lumen arrangement 103 includes an electrical conductor arrangement, a pressurizable lumen arrangement, and a guidewire lumen 101 dimension to receive a guidewire 110. The guidewire 110 can be used by the clinician to access a patient's venous or arterial system, locate a target vessel, such as the patient's renal artery, and advanced the therapy device 104 into the lumen of the target vessel. The proximal end of the shaft 102 is fluidly and electrically coupled to an external control system via the lumen arrangement 103, an embodiment of which is described hereinbelow with reference to Figure 11.

In the embodiment shown in Figure 4, the lumen arrangement 103 includes a supply lumen 118 through which a thermal transfer fluid is supplied to the therapy device 104 from an external source coupled to a proximal end of the shaft 102. The lumen arrangement 103 also includes a return lumen 119, through which spent thermal transfer fluid is returned to the proximal end of the shaft 102. According to some embodiments, the cooling arrangement 106 can include a phase-change cryothermal mechanism, a simpler heat exchanger system with liquid coolant, or a solid-state thermoelectric cooling device, for example. Depending on the particular cooling arrangement employed, one or both of the supply and return lumen's 118, 119 may or may not be required. Various cooling elements and support, connection, and control arrangements and methodologies that can be adapted for use in embodiments of the present disclosure are disclosed in commonly owned U.S. Patent No. 7,238,184 and U.S. Patent Application No. 13/157,844 filed June 10, 2011.

According to various embodiments, the electrodes 108 are cooled using a thermal transfer fluid supplied by an external coolant source and transported through the lumen arrangement 103 of the shaft 102. A variety of thermal transfer fluids may be employed, including cold saline or cold saline and ethanol mixture, Freon or other fluorocarbon refrigerants, nitrous oxide, liquid nitrogen, and liquid carbon dioxide, for example. The cooling arrangement 106 of the therapy unit 104 may include a tube (e.g., a cryoprobe), lumen, manifold, and/or a balloon arrangement through which the thermal transfer fluid passes. The cooling arrangement 106 may be integral or separate from the expandable housing 121. In some configurations, the cooling arrangement 106 may be configured to cool a substantial portion of the housing wall, including locations where the electrodes 108 are mounted. In other configurations, the cooling arrangement 106 may be configured to cool only those portions of the housing wall where the electrodes 108 are mounted.

In accordance with various embodiments, the electrodes 108 are energized by a conductive thermal transfer fluid within the housing 121. An electrical conductor extends along the lumen arrangement of the shaft 102 and is in electrical communication with the conductive fluid. In some configurations, the electrical conductor is electrically coupled to an electrode 112 positioned on the shaft 102 within the housing 121. High frequency AC power is communicated to the electrodes 108 supported by the housing 121 via the electrical conductor, electrode 112, and electrically conductive fluid within the housing 121. Various embodiments may incorporate selected structural, electrical, thermal, and control features of the devices disclosed in the commonly owned U.S. Serial No. 13/188,677 on July 22, 2011, which claims priority to U.S. Provisional Application Nos. 61/411,795, filed on November 9, 2010, and 61/369,442, filed on July 30, 2010. In other embodiments, the electrodes 108 are energized by electrical conductors that couple each electrode 108 to a conductor arrangement of the shaft 102. The electrodes 108 can be connected to an external control system individually or in series.

In some embodiments, the thermal transfer fluid, when released inside the cooling arrangement 106 (e.g., a cryoballoon) via the supply lumen 118, undergoes a phase change that cools some or all of the housing 121 and each of the electrodes 108 by absorbing the latent heat of vaporization from the tissue surrounding the therapy unit 104, and by cooling of the vaporized gas as it enters a region of lower pressure inside the cooling arrangement 106 (the Joule-Thomson effect). As a result of the phase change and the Joule-Thompson effect, heat is extracted from the surroundings of the housing 121, thereby cooling at least the electrodes 108 (and other portions of the housing wall if desired) which are in contact with vessel wall tissue. In configurations where cooling is limited to the electrodes 108, a manifold can be implemented within the housing 121 or housing wall to transport thermal transfer fluid to and from the electrodes 108. The gas released inside the cooling arrangement 106 may be exhausted through the return lumen 119 of the shaft 102. The pressure inside the cooling arrangement 106 may be controlled by regulating one or both of a rate at which thermal transfer fluid is delivered and a rate at which the exhaust gas is extracted. The lumen 118, 119 of the lumen arrangement 103 which transport thermal transfer fluid are preferably lined with or otherwise incorporate insulation material(s) having appropriate thermal and mechanical characteristics suitable for a selected thermal transfer fluid.

Embodiments of the present invention may incorporate selected balloon, catheter, lumen, control, and other features of the devices disclosed in the following commonly owned U.S. patents and published patent applications: U.S. Patent Publication Nos. 2009/0299356, 2009/0299355, 2009/0287202, 2009/0281533, 2009/0209951, 2009/0209949, 2009/0171333, 2008/0312644, 2008/0208182, 2008/0058791 and 2005/0197668, and U.S. Patent Nos. 5868735, 6290696, 6648878, 6666858, 6709431, 6929639, 6989009, 7022120, 7101368, 7172589, 7189227, and 7220257. Embodiments of the present invention may incorporate selected balloon, catheter, and other features of the devices disclosed in U.S. Patent Nos. 6355029, 6428534, 6432102, 6468297, 6514245, 6602246, 6648879, 6786900, 6786901, 6811550, 6908462, 6972015, and 7081112.

In various embodiments, the cooling arrangement 106 can include one or more thermoelectric elements configured to thermally couple to the wall of the housing 121 at or near the electrodes 108 and operate in a hypothermic mode. The thermoelectric elements preferably comprise solid-state thermoelectric elements, such as Peltier elements. Various Peltier-effect elements and support, connection, and control arrangements and methodologies that can be adapted for use in embodiments of the present invention are disclosed in commonly owned U.S. Patent No. 7,238,184.

In some embodiments, for example, the expandable housing 121 includes or is constructed as a balloon which is fluidly coupled to the lumen arrangement 103 and transformable between a low-profile introduction configuration and a larger-profile deployed configuration. The housing 121 is typically constructed from polymeric material, and preferably has a diameter dimensioned to fit within a target vessel, such as a renal artery of an average patient. It is understood that different models of ablation catheters 100 can be constructed each having specific housing configurations and dimensions appropriate for a given population of patients. In some embodiments, the housing 121 may comprise an expandable element, such as a pressurizable balloon or a mechanically expandable arrangement (e.g., an expandable-collapsible mesh structure). Use of such an expandable element in the construction of the housing 121 allows for use of a common housing design for a population of patients having varying anatomy. In accordance with various embodiments in which a pressurizable balloon is used in the construction of the housing 121, a thermal transfer fluid may be used for pressurizing the balloon and cooling of vessel tissue and the electrodes 108.

The balloon 121 includes a wall configured to contact an inner wall of a target vessel when in its deployed configuration. A multiplicity of ablation electrodes 108 are supported by the balloon wall and are preferably arranged in a predefined pattern. The electrodes 108 may, for example, be arranged to form one or more circumferential patterns. By way of further example, the electrodes 108 may be arranged to form a helical or spiral pattern. The ablation electrodes 108 are configured to deliver electrical energy sufficient to ablate target tissue located adjacent to the target vessel when the balloon 121 is in its deployed configuration. All or at least part of the cooling arrangement 106 is encompassed by the balloon 121.

As discussed previously, the cooling arrangement 106 is configured to cool at least the electrodes 108 during ablation, such that a location at which steady-state ablative heating begins is translated from an electrode-tissue interface at the inner vessel wall to a location a predetermined distance away from electrode-tissue interface. In some embodiments, the cooling arrangement is configured to cool the electrodes 108 such that the steady-state ablative heating begins at a distance of about 0.5 mm to about 1 mm from the electrodes 108 (away from the electrode-tissue interface and towards target tissue). In other embodiments, the location at which steady-state ablative heating begins is translated from the electrode-tissue interface to a distance of about 1 mm.

As is shown in Figure 5, one or more temperature sensors 115 can be situated on the therapy device 104 to provide for temperature sensing at or near the electrodes 108 and/or the target vessel wall. In the embodiment shown in Figure 5, each of the electrodes 108 is mounted to the wall of the housing 121 along with a corresponding temperature sensor 115. In some configurations, the electrodes 108 can be mounted so as to directly contact the corresponding temperature sensor 115. In such a configuration, the temperature of each electrode 108 can be individually monitored and energy delivered from each electrode 108 can be individually controlled. Although in some embodiments it may be desirable to connect the electrodes 108 in series to a common conductor, it may be more desirable to provide individual connectivity with at least some of the electrodes 108, allowing for selective energizing of the electrodes 108.

With further reference to the embodiment shown in Figure 5, the therapy unit 104 incorporates a cooling arrangement 106 in which cooling of the housing wall and electrodes 108 is provided by blood passing through the target vessel within which the therapy unit 104 is deployed. The embodiment shown in Figure 5 includes a cooling channel 150 that extends through a longitudinal portion of the housing 121. The cooling channel 150 includes an inlet 152 which is configured to divert blood flowing through the target vessel into the cooling channel 150. The cooling channel 150 further includes an outlet 154 through which heated blood returns to the target vessel. Although the cross-sectional illustration of the embodiment shown in Figure 5 shows a single cooling channel 150, it is understood that two or more cooling channels 150 may be incorporated into the housing 121 (e.g., between 2 and 6).

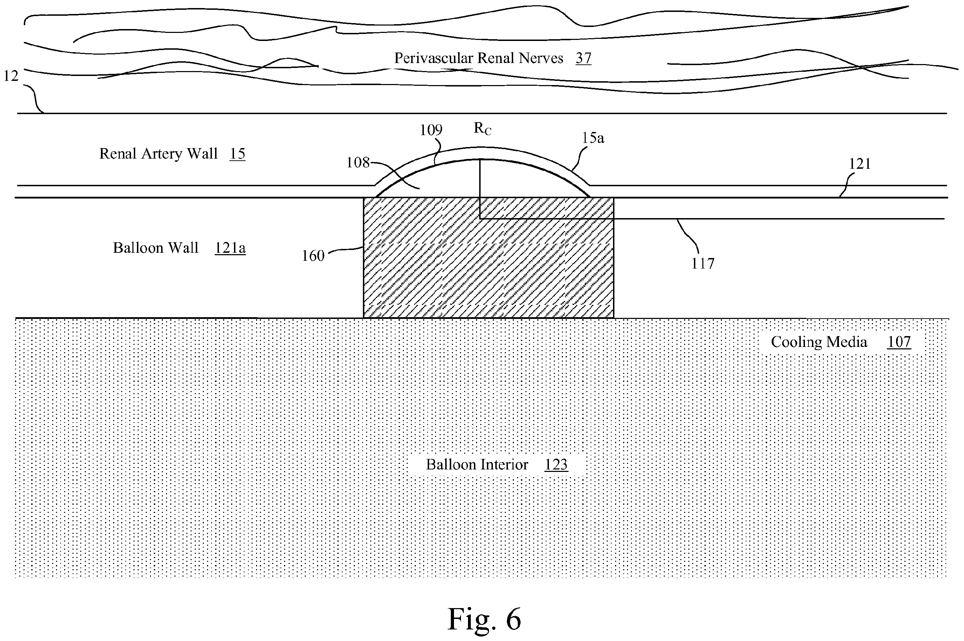

Figure 6 illustrates a portion of a therapy unit 104 of an ablation catheter 100 positioned within a lumen of a renal artery 12 in its deployed configuration. More particularly, Figure 6 shows an ablation electrode 108 supported by the wall 121a of a balloon 121. According to some embodiments, an electrical conductor 117 is connected to the electrode 108 and extends within or along the balloon wall 121a. The conductor 117 extends along the length of the shaft 102 and terminates at a coupling at the proximal end of the ablation catheter 100. The electrical conductor 117 may alternatively be disposed in an interior or exterior lumen provided along the interior or exterior of the balloon 121. In other embodiments, the electrical conductor 117 terminates at a location within the balloon other than at the electrode(s) 108. For example, and as previously discussed with reference to the embodiment of Figure 4, an electrode can be situated on the shaft of the balloon structure and coupled to the electrical conductor 117 which extends along the length of the catheter's shaft. High frequency alternating current is conducted from the shaft electrode to the electrode(s) 108 via an electrically conductive thermal transfer fluid within the balloon 121.

The electrode 108 is shown mounted to the outer surface of the balloon wall 121a. In the embodiment shown in Figure 6, a thermal conductor 160 is affixed to the balloon wall 121a and can serve as a base structure to facilitate mounting of the electrode 108 to the balloon wall 121a. The thermal conductor 160 preferably enhances the transfer of thermal energy between the cooling media 107 and the electrode 108. Although the thermal conductor 160 is shown extending through the thickness of the balloon wall 121a, the thermal conductor 160 can extend into the balloon interior 123 or only partially within the balloon wall 121a. The thermal conductor 160 may be fabricated using a matrix of polymeric and conductive material, which provides for pliancy of the thermal conductor 160.

As is further shown in the embodiment of Figure 6, the electrode 108 includes a protuberance 109 defining a tissue contacting surface which serves to compress a portion of the renal artery wall 15 when the balloon 121 is in its pressurized deployed configuration. The protuberance 109 of the electrode 108 is shown to have a continuous curved shape. The pressurized balloon 121 forces the protuberance 109 of the electrode 108 against the renal artery wall 15, thereby compressing a portion of the renal artery wall 15 shown as compression region, R_{C}, surrounding the electrode protuberance 109.

Compressing the renal artery wall 15 using the electrode protuberance 109 reduces the width of a renal artery wall portion 15a in the area of the electrode 108 and shortens the distance between the electrode 108 and target tissue (e.g., perivascular renal nerves 37). The effective reduction in the distance between the electrode 108 and the perivascular renal nerves 37 adjacent the renal artery 12 can facilitate a reduction in the amount of electrical energy needed to ablate the perivascular renal nerve tissue, due to a reduced amount of tissue through which the electrical energy must pass. A reduction in the amount of electrical energy needed to ablate target tissue can result in a reduction in the total amount of heat generated during ablation, resulting in reduced risk of thermal injury to non-targeted tissue.

A significant reduction in the total heat generated within the renal artery wall 15 is realized by cooling the electrode 108 during ablation. As previously discussed, it has been found that cooling the electrode 108 using a cooling arrangement of the type discussed herein advantageously translates outwardly the location at which steady-state ablative heating begins a predetermined distance away (i.e., a predetermined distance away from the tissue-electrode interface defined between the electrode protuberance 109 and adjacent renal artery wall tissue and in a direction of the perivascular renal nerve tissue).

The magnitude of this translation may be influenced by a number of factors including the amount of power delivered to the electrode 108, shape, size, and material of the electrode protuberance 109, temperature of the electrode 108 during cooling, renal artery wall thickness, the amount of renal artery wall compression, and other properties of the renal artery and neighboring tissue, among others. In general, the magnitude of this translation can range between about 0.5 mm to about 1 mm. An appreciable reduction in thermal injury to the artery wall is realizable when the start of steady-state heating is translated about 0.5 mm from the electrode-tissue interface, with further reductions in artery wall injury being realized until a translation of about 1 mm is achieved. Because artery anatomy differs between individual patients, it is understood that the range of about 0.5 mm to about 1 mm is an estimated range in which a beneficial reduction in thermal injury to the artery wall can be achieved for most patients. This range may be greater or smaller by about +/- 0.1 mm, +/- 0.2 mm, or +/- 0.3 mm (for one or both extremes of the range), for example, for some patients. In qualitative terms, the magnitude of this translation is preferably such that target tissue is effectively ablated while non-targeted tissue is subject to an acceptable level of thermal injury (e.g., little or no permanent thermal injury).

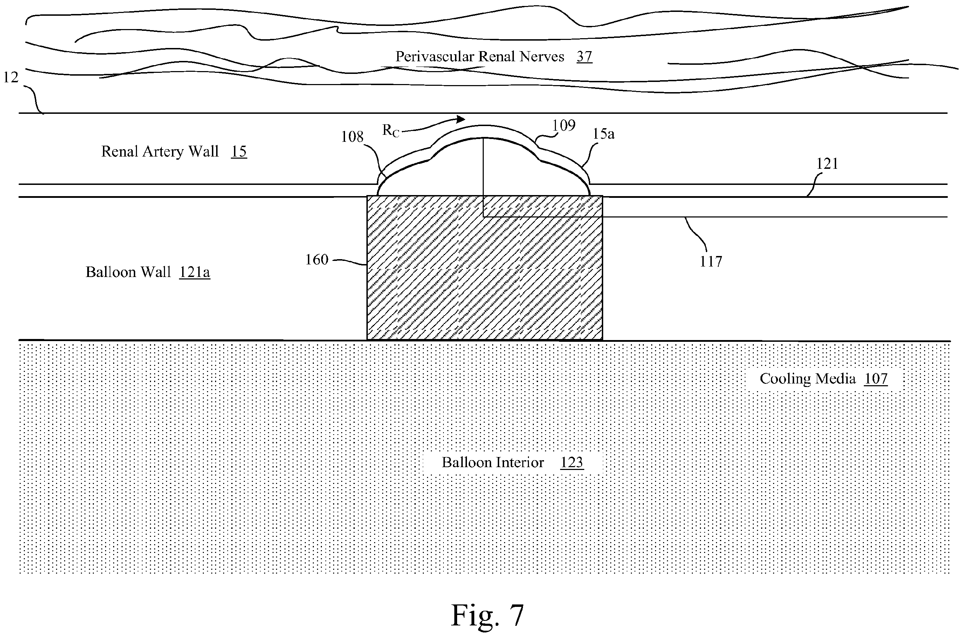

Figure 7 shows a portion of the therapy unit 104 of an ablation catheter 100 positioned within a lumen of the renal artery 12 in its deployed configuration. The therapy unit 104 shown in Figure 7 is similar in most aspects to that shown in Figure 6, but differs in terms of the shape of the protuberance 109 of ablation electrode 108. Whereas the protuberance 109 of the electrode 108 in the embodiment of Figure 6 has a continuous curved shape, the protuberance 109 of the electrode 108 in the embodiment of Figure 7 has a complex curved shape. The profile of the protuberance 109 of the electrode in Figure 7 includes a discontinuity such that a lower portion of the electrode 108 has a more gradual slope relative to that of an upper portion of the electrode 108. The smaller radius of curvature of the upper portion of the electrode 108 serves to concentrate greater compressive force at the tip of the electrode 108 when compared to an electrode 108 having a continuous curved shape. The protuberance 109 of Figure 7 provides for increased compression of the renal artery wall portion 15a in contact with the electrode 108, resulting in a further reduction in separation distance between the electrode 108 and the target tissue (perivascular renal nerves 37) located adjacent to the renal artery 12.

It is understood that, in some embodiments, the electrodes 108 can be flush or nearly flush with the outer surface of the housing 121 of the therapy unit 104. Many of the attributes described herein with regard to cooled electrodes 108 having protuberances 109 can be realized when using flush or near-flush mounted ablation electrodes 108, but with some degree of reduced benefits.

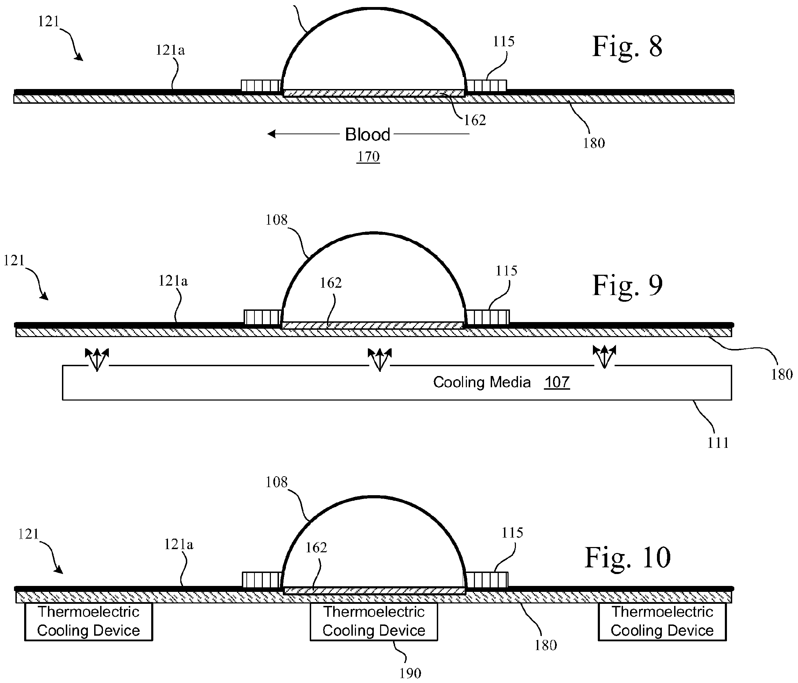

Figures 8-10 show a portion of a therapy unit 104 of an ablation catheter 100 including different cooling arrangements incorporated into a balloon 121 in accordance with various embodiments of the disclosure. Figure 8 shows an embodiment in which blood passing through the vessel is used for cooling within the therapy unit 104 (see, e.g., embodiment of Figure 5). The sectional view of Figure 8 shows an ablation electrode 108 supported by the wall 121a of a balloon 121 of the therapy unit 104. The electrode 108 is mounted on or otherwise coupled to a temperature sensor 115. In some embodiments, an inner surface of the balloon wall 121a is lined with a thermally conductive layer of material 180, such as a metallic foil layer. The thermally conductive layer 180 serves to enhance the transfer of thermal energy from the blood 170 flowing through the vessel, thereby enhancing cooling of the electrode 108. It is noted that the configuration and material of the temperature sensor 115 may be selected to also enhance thermal energy transfer between the electrode 108 and the blood 170. For example, the temperature sensor 115 may be constructed as a heat sink. An electrical insulator 162 may be used to electrically insulate the electrode 108 from the thermally conductive layer 180.

Figure 9 shows an embodiment in which a cooling media 107 is supplied to the balloon 121 via a manifold 111. The embodiment shown in Figure 9 is essentially the same as that shown in Figure 8, but differs in terms of the cooling arrangement configuration. In Figure 9, the manifold 111 disperses the cooling media 107 within the balloon 121 as either a gas or a liquid depending on the configuration of the cooling arrangement (e.g., a phase-change or heat exchange cooling arrangement). As previously discussed, the manifold 111 can be configured to disperse the cooling media 107 to all or most of the balloon wall 121a or only to those portions where electrodes 108 are mounted, in which case the conductive metallic layer 180 can either be excluded or limited to balloon wall regions adjacent the electrodes 180.

Figure 10 shows an embodiment in which thermoelectric cooling devices 190 are incorporated in the cooling arrangement. As shown in Figure 10, one or more thermoelectric cooling devices 190 are coupled to an inner surface of the balloon wall 121a. The thermoelectric cooling devices 190, for example, can be mounted to the thermally conductive layer 180, which provides for lateral conduction of thermal energy along the balloon wall 121a. In some configurations, a patch 180 of conductive metallic material can be affixed to the inner surface of the balloon wall 121a under individual electrodes 180 or under a subset of the electrodes 180. A thermoelectric cooling device 190 can be affixed to each of the conductive metallic material patches 180. The thermoelectric cooling devices 190 are preferably individually controlled during ablation, allowing for enhanced control of the temperature at each electrode 108. It is understood that a therapy unit 104 can incorporate more than one cooling arrangement of a type described herein, and that the cooling arrangements may be modified based on the application of a given therapy unit 104.

Referring now to Figure 11, there is shown a system 300 for ablating tissue that influences sympathetic renal nerve activity in accordance with various embodiments. The system 300 shown in Figure 11 includes a therapy device 104 provided at the distal end of a therapy catheter 100 deployed within a patient's renal artery 12. The therapy catheter 100 includes a flexible shaft 102 within which a lumen arrangement 103 is provided. The shaft 102 is preferably sufficient in length to reach a patient's renal artery 12 from a percutaneous access location 129. It may be desirable to use an external sheath 105 to facilitate delivery of the therapy device 104 into the renal artery 12. The catheter shaft 102 may include a distal hinge 356 that facilitates navigation of a near 90° turn into the renal artery 12 from the aorta 20.

The therapy device 104 includes an electrode arrangement and a cooling arrangement of a type previously described. The electrode arrangement is electrically coupled to an external radiofrequency (RF) generator 320. A power control 322 and timing control 324 provide for automatic or semi-automatic control of electrical energy delivery from the therapy unit 104. The cooling arrangement of the therapy device 104 is shown fluidly coupled to a coolant source 340. A temperature control 324 is preferably coupled to one or more temperature sensors provided at the therapy device 104. The temperature control 324 generates temperature signals which are used by the RF generator 320 and coolant source 340 to adjust (automatically via a processor of the system 300 or semi-automatically) power delivered to the ablation electrodes 108 and thermal transfer fluid delivered and/or removed to/from the cooling arrangement of the therapy device 104.

A pump system 341 is shown coupled to the coolant source 340. The pump system 341 is coupled to a fluid reservoir system which may be configured to store a variety of cryogens, such as cold saline or cold saline and ethanol mixture, Freon or other fluorocarbon refrigerants, nitrous oxide, liquid nitrogen, and liquid carbon dioxide, for example.

Various embodiments disclosed herein are generally described in the context of ablation of perivascular renal nerves for control of hypertension. It is understood, however, that embodiments of the disclosure have applicability in other contexts, such as performing ablation from within other vessels of the body, including other arteries, veins, and vasculature (e.g., cardiac and urinary vasculature and vessels), and other tissues of the body, including various organs (e.g., the prostate for BPH ablation).

It is to be understood that even though numerous characteristics of various embodiments have been set forth in the foregoing description, together with details of the structure and function of various embodiments, this detailed description is illustrative only, and changes may be made in detail, especially in matters of structure and arrangements of parts illustrated by the various embodiments to the full extent indicated by the broad general meaning of the terms in which the appended claims are expressed.

## Claims

1. An apparatus, comprising:
a catheter arrangement (100) comprising a flexible shaft (102);
a balloon (121) disposed at a distal end of the shaft (102) and configurable for deployment within a target vessel of the body;
a plurality of ablation electrodes (108) supported by a wall of the balloon (121), the ablation electrodes (108) configured to deliver electrical energy sufficient to ablate target tissue proximate a wall of the target vessel when the balloon (121) is in a deployed configuration; and
a cooling arrangement (106) encompassed at least in part by the balloon (121) and configured to provide cooling to at least the electrodes (108) during ablation such that a location at which steady-state ablative heating begins is translated from an electrode-tissue interface at the target vessel wall to a location a predetermined distance away from the electrode-tissue interface, wherein the cooling arrangement (106) comprises:
a phase-change cryothermal apparatus configured to receive a liquid cooling media and output spent gas resulting from the cryothermal phase-change; or
a heat exchange apparatus configured to receive a cooled liquid cooling media and output spent liquid cooling media; or
one or more solid-state thermoelectric cooling devices; and
wherein the apparatus further comprises an electrical conductor extending along the shaft (102) and arranged to be in electrical communication with a conductive fluid within the balloon (121), such that the electrodes (108) can be energized by the conductive fluid.

2. The apparatus of claim 1, comprising one or more temperature sensors supported by the balloon wall and configured to sense a temperature at or proximate the target vessel wall during ablation, wherein the cooling arrangement is configured to cool the electrodes based on the sensed temperature such that the steady-state ablative heating begins at a distance of at least about 0.5 mm away from the electrodes.

3. The apparatus of claim 1, comprising one or more temperature sensors supported by the balloon wall and configured to sense a temperature at or proximate the target vessel wall during ablation, wherein the cooling arrangement is configured to cool the electrodes based on the sensed temperature such that the steady-state ablative heating begins at a distance of about 0.5 mm to about 1 mm away from the electrodes.

4. The apparatus of claim 1, wherein the electrodes are arranged on the balloon wall to define a spiral pattern or one or more circumferential patterns.

5. The apparatus of claim 1, wherein each of the ablation electrodes comprises a protuberance defining a tissue contacting surface which serves to compress a portion of the target vessel wall and deliver the electrical energy through the compressed portion of the target vessel wall.

6. The apparatus of claim 1, wherein each of the electrodes has a continuous curved shape or a complex curved shape having a protuberance (109) with a profile that includes a discontinuity such that a lower portion of the electrode (108) has a more gradual slope relative to that of an upper portion of the electrode (108).

7. The apparatus of claim 1, comprising one or more temperature sensors supported by the balloon wall and configured to sense a temperature at or proximate the target vessel wall during ablation.

8. The apparatus of claim 1, wherein at least portions of an inner wall of the balloon adjacent the electrodes comprise a layer of thermally conductive material configured to enhance thermal energy transfer between the cooling arrangement and the electrodes during ablation.

9. The apparatus of claim 1, comprising an external system coupled to the proximal end of the catheter arrangement, the system configured to control power delivered to the electrodes and coolant delivered to the cooling arrangement.

10. The apparatus according to any of the preceding claims, wherein:
the shaft has a length sufficient to access a patient's renal artery relative to a percutaneous access location; and
the cooling arrangement is configured to provide cooling to at least the electrodes during ablation of perivascular renal nerves adjacent the electrodes such that a location at which steady-state ablative heating begins is translated from an electrode-tissue interface at a wall of the renal artery to a location a predetermined distance away from the electrode-tissue interface.

11. The apparatus according to any of the preceding claims, wherein:
the shaft has a proximal end, a distal end, a length, and a lumen arrangement extending between the proximal and distal ends, the length of the shaft sufficient to access a patient's renal artery relative to a percutaneous access location;
the balloon is dimensioned for deployment within the renal artery and transformable between a low-profile introduction configuration and a larger-profile deployed configuration, the balloon fluidly coupled to the lumen arrangement and comprising a wall configured to contact a wall of the renal artery when in the deployed configuration;
the plurality of ablation electrodes are configured to deliver electrical energy sufficient to ablate perivascular renal nerves adjacent the renal artery when the balloon is in the deployed configuration; and
the cooling arrangement is configured to provide cooling to at least the electrodes during ablation such that a location at which steady-state ablative heating begins is translated from an electrode-tissue interface at the renal artery wall to a location a predetermined distance away from the electrode-tissue interface.

12. The apparatus of claim 11, wherein the lumen arrangement comprises a guide lumen dimensioned to receive a guidewire.

## Patentansprüche

1. Vorrichtung, die aufweist:
eine Katheteranordnung (100), die einen flexiblen Schaft (102) aufweist;
einen Ballon (121), der an einem distalen Ende des Schafts (102) angeordnet und zur Entfaltung in einem Zielgefäß des Körpers konfigurierbar ist;
mehrere Ablationselektroden (108), die durch eine Wand des Ballons (121) gehalten werden, wobei die Ablationselektroden (108) konfiguriert sind, elektrische Energie abzugeben, die ausreicht, um Zielgewebe benachbart einer Wand des Zielgefäßes zu ablatieren, wenn sich der Ballon (121) in einer entfalteten Konfiguration befindet; und
eine Kühlanordnung (106), die mindestens teilweise durch den Ballon (121) umgeben und konfiguriert ist, mindestens für die Elektroden (108) während der Ablation eine Kühlung bereitzustellen, so dass eine Stelle, an der eine dauernde ablative Erwärmung beginnt, von einer Elektroden-Gewebe-Grenzfläche an der Zielgefäßwand zu einer Stelle in einem vorgegebenen Abstand von der Elektroden-Gewebe-Grenzfläche entfernt verschoben wird, wobei die Kühlanordnung (106) aufweist:
eine cryothermische Phasenwechselvorrichtung, die konfiguriert ist, ein flüssiges Kühlmedium aufzunehmen und verbrauchtes Gas abzugeben, das aus dem cryothermischen Phasenwechsel resultiert; oder
eine Wärmetauschervorrichtung, die konfiguriert ist, ein abgekühltes flüssiges Kühlmedium aufzunehmen und ein verbrauchtes flüssiges Kühlmedium abzugeben; oder
eine oder mehrere thermoelektrische Festkörperkühlvorrichtungen; und
wobei die Vorrichtung ferner einen elektrischen Leiter aufweist, der sich längs des Schafts (102) erstreckt und eingerichtet ist, in einer elektrischen Verbindung mit einem leitfähigen Fluid innerhalb des Ballons (121) zu stehen, so dass die Elektroden (108) durch das leitfähige Fluid erregt werden können.

2. Vorrichtung nach Anspruch 1, die einen oder mehrere Temperatursensoren aufweist, die durch die Ballonwand gehalten werden und konfiguriert sind, während der Ablation eine Temperatur an oder benachbart zur Zielgefäßwand abzutasten, wobei die Kühlanordnung konfiguriert ist, die Elektroden beruhend auf der abgetasteten Temperatur zu kühlen, so dass die dauernde ablative Erwärmung in einem Abstand von mindestens etwa 0,5 mm entfernt von den Elektroden beginnt.

3. Vorrichtung nach Anspruch 1, die einen oder mehrere Temperatursensoren aufweist, die durch die Ballonwand gehalten werden und konfiguriert sind, während der Ablation eine Temperatur an oder benachbart zur Zielgefäßwand abzutasten, wobei die Kühlanordnung konfiguriert ist, die Elektroden beruhend auf der abgetasteten Temperatur zu kühlen, so dass die dauernde ablative Erwärmung in einem Abstand von etwa 0,5 mm bis etwa 1 mm entfernt von den Elektroden beginnt.

4. Vorrichtung nach Anspruch 1, wobei die Elektroden so an der Ballonwand angeordnet sind, dass sie ein Spiralmuster oder ein oder mehrere Umfangsmuster bilden.

5. Vorrichtung nach Anspruch 1, wobei jede der Ablationselektroden einen Vorsprung aufweist, der eine Gewebekontaktfläche definiert, die dazu dient, einen Abschnitt der Zielgefäßwand zusammenzudrücken und die elektrische Energie durch den zusammengedrückten Abschnitt der Zielgefäßwand abzugeben.

6. Vorrichtung nach Anspruch 1, wobei jede der Elektroden eine kontinuierliche gekrümmte Form oder eine komplexe gekrümmte Form aufweist, die einen Vorsprung (109) mit einem Profil aufweist, der eine Unstetigkeit aufweist, so dass ein unterer Abschnitt der Elektrode (108) eine sachtere Neigung relativ zu der des oberen Abschnitts der Elektrode (108) aufweist.

7. Vorrichtung nach Anspruch 1, die einen oder mehrere Temperatursensoren aufweist, die durch die Ballonwand gehalten werden und konfiguriert sind, während der Ablation eine Temperatur an oder benachbart zur Zielgefäßwand abzutasten.

8. Vorrichtung nach Anspruch 1, wobei mindestens Abschnitte einer Innenwand des Ballons benachbart zu den Elektroden eine Schicht aus einem wärmeleitfähigen Material aufweisen, die konfiguriert ist, den Wärmeenergietransfer zwischen der Kühlanordnung und den Elektroden während der Ablation zu verbessern.

9. Vorrichtung nach Anspruch 1, die ein externes System aufweist, das mit dem proximalen Ende der Katheteranordnung gekoppelt ist, wobei das System konfiguriert ist, die Leistung, die an die Elektroden abgegeben wird, und das Kühlmittel, das an die Kühlanordnung abgegeben wird, zu regeln.

10. Vorrichtung nach einem der vorhergehenden Ansprüche, wobei:
der Schaft eine Länge aufweist, die ausreichend ist, bezüglich einer perkutanen Zugangsstelle einen Zugang zu einer Nierenarterie eines Patienten zu erhalten; und
die Kühlanordnung konfiguriert ist, während der Ablation von perivaskulären Nierennerven benachbart zu den Elektroden mindestens für die Elektroden eine Kühlung bereitzustellen, so dass eine Stelle, an der die dauernde ablative Erwärmung beginnt, von einer Elektroden-Gewebe-Grenzfläche an einer Wand der Nierenarterie zu einer Stelle in einem vorgegebenen Abstand von der Elektroden-Gewebe-Grenzfläche entfernt verschoben wird.

11. Vorrichtung nach einem der vorhergehenden Ansprüche, wobei:
der Schaft ein proximales Ende, ein distales Ende, eine Länge und eine Lumenanordnung aufweist, die sich zwischen dem proximalen und distalen Ende erstreckt, wobei die Länge des Schafts ausreichend ist, bezüglich einer perkutanen Zugangsstelle einen Zugang zu einer Nierenarterie eines Patienten zu erhalten;
der Ballon zur Entfaltung innerhalb der Nierenarterie bemessen ist und zwischen einer Einführungskonfiguration mit niedrigem Profil und einer entfalteten Konfiguration mit größerem Profil umwandelbar ist, wobei der Ballon fluidisch mit der Lumenanordnung gekoppelt ist und eine Wand aufweist, die konfiguriert ist, in der entfalteten Konfiguration eine Wand der Nierenarterie zu berühren;
die mehreren Ablationselektroden konfiguriert sind, elektrische Energie abzugeben, die ausreicht, um perivaskuläre Nierennerven benachbart zur Nierenarterie zu ablatieren, wenn sich der Ballon in der entfalteten Konfiguration befindet; und
die Kühlanordnung konfiguriert ist, mindestens für die Elektroden während der Ablation eine Kühlung bereitzustellen, so dass eine Stelle, an der eine dauernde ablative Erwärmung beginnt, von einer Elektroden-Gewebe-Grenzfläche an der Nierenarterienwand zu einer Stelle in einem vorgegebenen Abstand von den Elektroden-Gewebe-Grenzfläche entfernt verschoben wird.

12. Vorrichtung nach Anspruch 11, wobei die Lumenanordnung ein Führungslumen aufweist, das bemessen ist, einen Führungsdraht aufzunehmen.

## Revendications

1. Appareil, comprenant :
un dispositif de cathéter (100) comprenant une tige flexible (102);
un ballonnet (121) disposé à une extrémité distale de la tige (102) et configurable pour être déployé à l'intérieur d'un vaisseau cible du corps ;
une pluralité d'électrodes d'ablation (108) supportées par une paroi du ballonnet (121),
lesdites électrodes d'ablation (108) étant prévues pour diffuser une énergie électrique suffisante pour réaliser l'ablation d'un tissu cible à proximité d'une paroi du vaisseau cible quand le ballonnet (121) est en configuration déployée ; et
un dispositif de refroidissement (106) enveloppé au moins en partie par le ballonnet (121) et prévu pour refroidir au moins les électrodes (108) pendant l'ablation, de manière à transférer un emplacement où commence un chauffage ablatif permanent, d'une interface électrode-tissu sur la paroi de vaisseau cible vers un emplacement situé à une distance définie de l'interface électrode-tissu, ledit dispositif de refroidissement (106) comprenant :
un dispositif cryothermique à changement de phase prévu pour recevoir un agent de refroidissement liquide et refouler un gaz utilisé résultant du changement de phase cryothermique ; ou
un dispositif échangeur de chaleur prévu pour recevoir un agent de refroidissement liquide refroidi et refouler un agent de refroidissement liquide utilisé ; ou
un ou plusieurs dispositifs de refroidissement thermoélectriques solides ; et
où ledit appareil comprend en outre un conducteur électrique s'étendant le long de la tige (102) et disposé de manière à être en liaison électrique avec un fluide conducteur à l'intérieur du ballonnet (121), pour permettre l'énergisation des électrodes (108) par le fluide conducteur.

2. Appareil selon la revendication 1, comprenant un ou plusieurs capteurs de température supportés par la paroi de ballonnet et prévus pour détecter une température sur la paroi de vaisseau cible ou dans l'environnement de celle-ci pendant l'ablation, le dispositif de
refroidissement étant prévu pour refroidir les électrodes sur la base de la température détectée, de sorte que le chauffage ablatif permanent commence à une distance d'au moins 0,5 mm des électrodes.

3. Appareil selon la revendication 1, comprenant un ou plusieurs capteurs de température supportés par la paroi de ballonnet et prévus pour détecter une température sur la paroi de vaisseau cible ou dans l'environnement de celle-ci pendant l'ablation, le dispositif de refroidissement étant prévu pour refroidir les électrodes sur la base de la température détectée, de sorte que le chauffage ablatif permanent commence à une distance sensiblement comprise entre 0,5 mm et 1 mm des électrodes.

4. Appareil selon la revendication 1, où les électrodes sont disposées sur la paroi de ballonnet de manière à définir un motif en spirale ou un ou plusieurs motifs circonférentiels.

5. Appareil selon la revendication 1, où chaque électrode d'ablation présente une saillie définissant une surface de contact de tissu servant à comprimer une partie de la paroi de vaisseau cible et à diffuser l'énergie électrique par la partie de paroi de vaisseau cible comprimée.

6. Appareil selon la revendication 1, où chaque électrode a une forme courbe continue ou une forme courbe complexe présentant une saillie (109) ayant un profil avec une discontinuité telle qu'une partie inférieure de l'électrode (108) présente une pente plus graduelle qu'une partie supérieure de l'électrode (108).

7. Appareil selon la revendication 1, comprenant un ou plusieurs capteurs de température supportés par la paroi de ballonnet et prévus pour détecter une température sur la paroi de vaisseau cible ou dans l'environnement de celle-ci pendant l'ablation.

8. Appareil selon la revendication 1, où au moins des parties d'une paroi intérieure du ballonnet adjacentes aux électrodes présentent une couche de matériau thermoconducteur prévue pour accroître le transfert d'énergie thermique entre le dispositif de refroidissement et les électrodes pendant l'ablation.

9. Appareil selon la revendication 1, comprenant un système externe relié à l'extrémité proximale du dispositif de cathéter, ledit système étant prévu pour commander la puissance fournie aux électrodes et le réfrigérant alimentant le dispositif de refroidissement.

10. Appareil selon l'une des revendications précédentes, où :
la tige a une longueur suffisante pour accéder à une artère rénale d'un patient depuis un emplacement d'accès percutané ; et
le dispositif de refroidissement est prévu pour refroidir au moins les électrodes pendant l'ablation de nerfs rénaux périvasculaires adjacents aux électrodes, de sorte qu'un emplacement où commence le chauffage ablatif permanent est transféré d'une interface électrode-tissu sur une paroi de l'artère rénale vers un emplacement à une distance définie de l'interface électrode-tissu.

11. Appareil selon l'une des revendications précédentes, où :
la tige a une extrémité proximale, une extrémité distale, une longueur et un dispositif de lumière s'étendant entre l'extrémité proximale et l'extrémité distale, la longueur de la tige étant suffisante pour accéder à une artère rénale d'un patient depuis un emplacement d'accès percutané;
le ballonnet est dimensionné pour un déploiement à l'intérieur de l'artère rénale et est transformable entre une configuration d'introduction à profil réduit et une configuration de déploiement à profil étendu, ledit ballonnet étant en liaison fluidique avec le dispositif de lumière et comprenant une paroi prévue pour contacter une paroi de l'artère rénale en configuration déployée ;
la pluralité d'électrodes d'ablation est prévue pour diffuser une énergie électrique suffisante pour réaliser l'ablation de nerfs rénaux périvasculaires adjacents à l'artère rénale quand le ballonnet est en configuration déployée ; et
le dispositif de refroidissement est prévu pour refroidir au moins les électrodes pendant l'ablation, de sorte qu'un emplacement où commence le chauffage ablatif permanent est transféré d'une interface électrode-tissu sur la paroi de l'artère rénale vers un emplacement à une distance définie de l'interface électrode-tissu.

12. Appareil selon la revendication 11, où le dispositif de lumière comprend une lumière-guide dimensionnée pour recevoir un fil-guide.
